# EUROPÄISCHE PATENTSCHRIFT

(11) **EP 0 995 440 B1**
(45) Veröffentlichungstag und Bekanntmachung des Hinweises auf die Patenterteilung: **21.09.2005**
(21) Anmeldenummer: 99115236.4
(22) Anmeldetag: 02.08.1999
(51) Int. Cl.: A61K 31/425, A61P 3/00

(54) **Verfahren zur Stiegerung des Blutglukosespiegels in Säugern**
Method to increase the blood, glucose level in mammals
Procédé pour augmenter le taux de glucose dans le sang des mammifères

(30) Priorität: 31.07.1998 DE 19834591
(43) Veröffentlichungstag der Anmeldung: 26.04.2000
(73) Patentinhaber: Prosidion Limited, Oxford, OX4 6LT (GB)
(72) Erfinder: Demuth, Hans-Ulrich, Dr., 06120 Halle/Saale (DE); Hoffmann, Torsten, Dr., 06120 Halle/Saale (DE); Kühn-Wache, Kerstin, 06120 Halle/Saale (DE); Rosche, Fred, Dr., 06120 Halle/Saale (DE)
(74) Vertreter: Blakey, Alison Jane

(56) Entgegenhaltungen:
- EP-A- 0 869 135
- WO-A-97/40832
- WO-A-98/19998
- WO-A-99/61431
- PEDERSON R A ET AL: "IMPROVED GLUCOSE TOLERANCE IN ZUCKER FATTY RATS BY ORAL ADMINISTRATION OF THE DIPEPTIDYL PEPTIDASE IV INHIBITOR ISOLEUCINE THIAZOLIDIDE" DIABETES,US,NEW YORK, NY, Bd. 48, Nr. 8, August 1998 (1998-08), Seiten 1253-1258, XP000853617 ISSN: 0012-1797
- STOECKEL, A. ET AL: "Competitive inhibition of proline specific enzymes by amino acid thioxopyrrolidides and thiazolidides" PEPT.: CHEM., STRUCT. BIOL., PROC. AM. PEPT. SYMP., 14TH (1996), MEETING DATE 1995, 709-710. EDITOR(S): KAUMAYA, PRAVIN T. P.;HODGES, ROBERT S. PUBLISHER: MAYFLOWER SCIENTIFIC, KINGSWINFORD, UK., XP000867517

## Beschreibung

Die Erfindung betrifft ein Verfahren, bei dem durch die Reduktion von Dipeptidyl Peptidase IV (DP IV)- bzw. DP IV-analoger Enzymaktivität im Blut eines Säugers durch Verabreichung von aktivitätsmindernden Effektoren das endogene (oder zusätzlich exogen verabreichte) glycogenolytisch wirksame Peptid Glucagon oder dessen Analoga durch DP IV- und DP IV-analoger Enzymaktivität vermindert abgebaut werden und damit die Konzentrationsabnahme dieses Peptidhormons bzw. seiner Analoga verringert bzw. verzögert wird.

In Folge dieser, durch die Wirkung von DP IV-Effektoren erzielten, erhöhten Stabilität des (endogen vorhandenen oder exogen zugeführten) Glucagons und seiner Analoga, die damit vermehrt für die glycogenolytische Stimulierung der Glucagon-Rezeptoren von insbesondere Leberzellen zur Verfügung stehen, verändert sich die Wirksamkeitsdauer von körpereigenem Glucagon, was eine Stimulierung des katabolen Kohlehydratstoffwechsels des behandelten Organismus nach sich zieht.

Als Resultat steigt der Blutzuckerspiegel über die für Hypoglycaemie charakteristische Glukosekonzentration im Serum des behandelten Organismus. Damit können Stoffwechselanomalien wie hypoglycaemische Zustände, die die Folge verminderter Glukosekonzentrationen im Blut sind, verhindert bzw. gemildert werden.

Neben Proteasen, die in unspezifische Proteolyse einbezogen sind, was letztlich den Abbau von Proteinen zu Aminosäuren bewirkt, kennt man regulatorische Proteasen, die an der Funktionalisierung (Aktivierung, Deaktivierung, Modulierung) von endogenen Peptidwirkstoffen beteiligt sind (Kirschke *et al.,* 1995; Kräusslich & Wimmer, 1987). Insbesondere im Zusammenhang mit der Immunforschung und der Neuropeptidforschung sind eine Reihe solcher sogenannten Konvertasen, Signalpeptidasen oder Enkephalinasen entdeckt worden (Gomez *et al.,* 1988; Ansorge *et al.,* 1991).

Aufgrund der Häufigkeit des Vorkommens der Aminosäure Prolin in einer Vielzahl von Peptidhormonen und den damit verbundenen Struktureigenschaften dieser Peptide wird für Prolinspezifische Peptidasen eine den Signalpeptidasen analoge Funktion diskutiert (Yaron & Naider, 1993; Walter *et al.,* 1980; Vanhoof *et al.,* 1995). Dabei bestimmt Prolin in diesen Peptiden durch seine besondere Struktur sowohl Konformation als auch Stabilität dieser Peptide, indem sie vor Abbau durch unspezifische Proteasen schützt (Kessler, 1982).

Enzyme, die hochspezifisch strukturverändernd auf Prolinhaltige Sequenzen einwirken (HIV-Protease, Cyclophylin u.a.) sind attraktive Ziele der aktuellen Wirkstoff-Forschung. Insbesondere für die nach dem Prolin spaltenden Peptidasen Prolyl Endopeptidase (PEP) und Dipeptidyl Peptidase IV (DP IV) konnten Beziehungen zwischen der Modulation der biologischen Aktivität von natürlichen Peptidsubstraten und deren selektiver Spaltung durch diese Enzyme wahrscheinlich gemacht werden. So nimmt man an, daß PEP eine Rolle beim Lernen bzw. im Gedächtnisprozeß spielt und DP IV in die Signalübertragung während der Immunantwort einbezogen ist (Ishiura *et al.,* 1990; Hegen *et al.,* 1990).

Ähnlich wie die außerordentliche Prolinspezifität dieser Enzyme wird ihre hohe Selektivität für die Aminosäure Alanin innerhalb typischer Erkennungsregionen in Substraten dieser Enzyme diskutiert, wonach Alanin-haltige Peptide ähnliche Konformationen einnehmen können wie strukturanaloge Prolinhaltige Peptide. Kürzlich wurden derartige Eigenschaften Alanin-haltiger Peptidketten durch Punktmutation (Austausch von Prolin gegen Alanin) nachgewiesen (Dodge & Scheraga, 1996).

DP IV- bzw. DP IV-analoge Aktivität (z. B. besitzt die cytosolische DP II eine der DP IV nahezu identische Substratspezifität) kommt im Blutkreislauf vor, wo sie hochspezifisch Dipeptide vom N-Terminus biologisch aktiver Peptide abspaltet, wenn Prolin oder Alanin die benachbarten Reste der N-terminalen Aminosäure in deren Sequenz darstellen. Aufgrund dieser Spaltstellenspezifität wird davon ausgegangen, daß dieses Enzym bzw. Analoga an der Regulation von Polypeptiden *in vivo* beteiligt sind (Vanhoof *et al.,* 1995).

Zu geringe Blutzuckerkonzentrationen können im menschlichen und tierischen Organismus zu pathologischen Zuständen führen. Insbesondere nach Unfällen kann es zu einem sogenannten hypoglycaemischen Schock kommen, was bei Patienten zu Heißhunger, Schweißausbrüchen, ja sogar zu Bewußtlosigkeit und Tod führen kann.

Es war daher eine Aufgabe der vorliegenden Erfindung, Mittel zur Verhinderung oder Milderung pathologischer Stoffwechsel-Anomalien von Säugerorganismen wie akuter oder chronischer Hypoglycaemie bereitzustellen.

Insbesondere war es eine Aufgabe der vorliegenden Erfindung, Mittel bereitzustellen, mit denen Kohlehydratreserven zum Beispiel der Leber rasch mobilisiert werden können.

Diese Aufgaben werden erfindungsgemäß durch die Verwendung von aktivitätsmindernden Effektoren der Dipeptidylpeptidase IV (DP IV) bzw. DP IV-analoger Enzymaktivität zur Herstellung eines Medikaments zur Reduktion von DP IV- bzw. DP IV-analoger Enzymaktivität Erhöhung des Blutzuckerspiegels in einem Säugerorganismus gelöst (Anspruch 1).

Es ist bereits bekannt, aktivitätsmindernde Effektoren der DP IV zur Erniedrigung des Blutzuckerspiegels von Säuger-Organismen einzusetzen. Dabei wird der Abbau von Incretinen, die den Glucoseabbau stimulieren, durch DP IV gestoppt.

Es ist daher besonders überraschend, daß aktivitätsmindernde Effektoren der DP IV bzw. DP IV-analoger Enzymaktivität zur Erhöhung des Blutzuckerspiegels eingesetzt werden können. Vermutlich beruht diese Wirkung auf folgenden Mechanismen:

Beim Glucosemetabolismus und -katabolismus im menschlichen und tierischen Körper kann grundsätzlich zwischen zwei Phasen unterschieden werden:
1. In der ersten Phase werden nach der Nahrungsaufnahme vermehrt Incretine ausgeschüttet (d.h. Hormone, die die Insulinsekretion des Pankreas stimulieren, wie Gastric Inhibitory Polypeptide 1-42 (GIP₁₋₄₂) und Glucagon-Like Peptide Amide-1 7-36 (GLP-1₇₋₃₆)), wodurch es zu einer vermehrten Insulinproduktion und in kausaler Folge zu einem verstärkten Abbau der durch Nahrungsaufnahme zugeführten Glucose kommt.
   Die Incretine sind jedoch Substrate der DP IV, da diese von den N-terminalen Sequenzen der Incretine die Dipeptide Tyrosinyl-Alanin bzw. Histidyl-Alanin *in vitro* und *in situ* abspalten kann (Mentlein *et al.,* 1993). Folglich kommt es bei Vorliegen von DP IV zu einem Abbau der Incretine, was wiederum zu einem verminderten Glucoseabbau führt.
   Durch die Inhibierung der DP IV- bzw. DP IV-analoger Enzymaktivität *in vivo* ist es daher möglich, einen übermäßigen Abbau der Incretine wirksam zu unterdrücken und folglich den Glucoseabbau zu steigern:
   - Die DP IV-Inhibierung führt zur Stabilisierung der Incretine,
   - die verlängerte Lebensdauer der Incretine im Blutkreislauf verstärkt ihre insulinotrope und insulinsensitivierende Wirkung,
   - die damit erhöhte und wirksamere Insulinausschüttung zieht eine verstärkte Glukosetoleranz nach sich, (Demuth *et al.,* 1996).

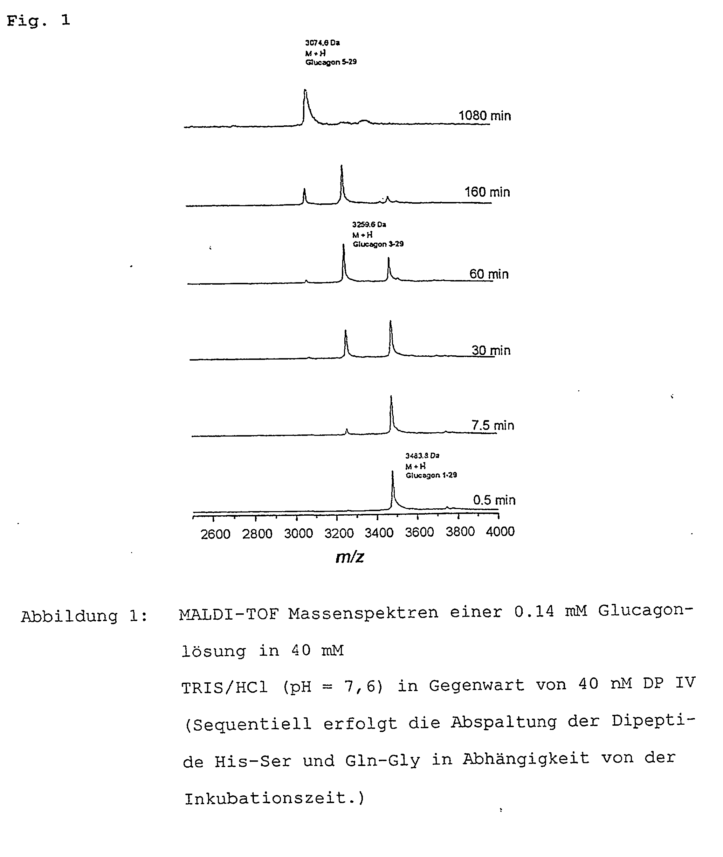

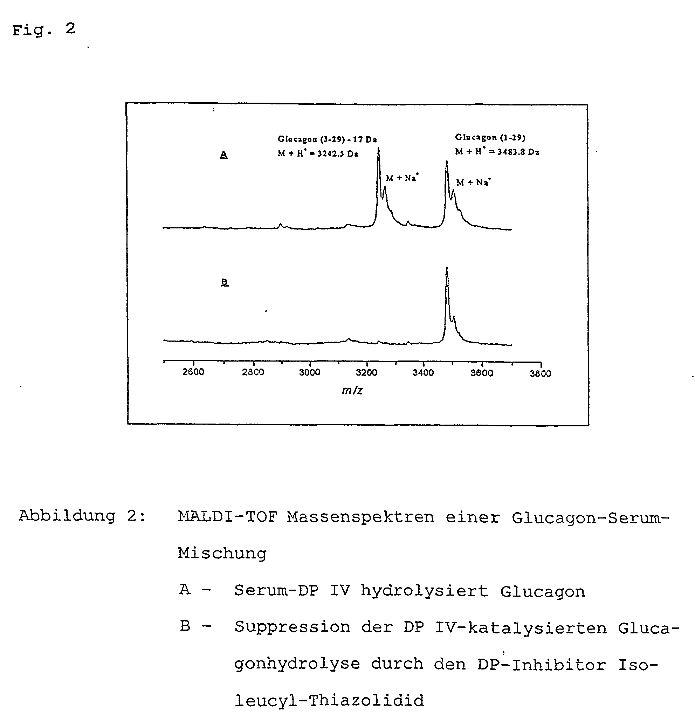

   An diabetischen Ratten wurde nachgewiesen, daß die entsprechenden DP IV-Inhibitoren zur Modulation des dargestellten Regelkreises wirkungsvoll eingesetzt werden können (Pederson *et al.,* 1998). Diese Phase dauert vom Zeitpunkt der Nahrungsaufnahme an etwa 120 Minuten.
   Nach Ablauf dieser sogenannten postprandialen Phase wird die Sekretion von Incretinen gestoppt und bereits vorhandene Incretine durch DP IV abgebaut. Dadurch sinkt die Insulinproduktion, was zu einem Ende des Glukoseabbaus führt.
2. Um die physiologisch notwendige Glukosekonzentration von etwa 5,5 mM/l zwischen den Nahrungsaufnahmen aufrecht zu erhalten, wird in der zweiten Phase eingelagertes Glykogen abgebaut, wozu Glucagon aus den pankreatischen A-Zellen freigesetzt wird. Glucagon weist also eine entgegengesetzte Wirkung zum Insulin und damit auch zu den Incretinen auf.
   Bei täglich drei Mahlzeiten steht der menschliche Körper demgemäß ca. 6 Stunden (3 x 120 Minuten) unter GLP-1/GIP-und Insulin-, aber 18 Stunden unter Glucagon-Kontrolle.
   Es ist festgestellt worden, daß DP IV endogen aus den selben sekretorischen Granulae der A-Zellen ausgeschüttet wird wie Glucagon und daß diese Ausschüttung zeitgleich mit der Glucagon-Ausschüttung bzw. dem Eintritt der Glucagon-Wirkung erfolgen kann. Erfindungsgemäß ist nun gefunden worden, daß Glucagon *in vitro* wie *in vivo* durch DP IV bzw. DP IV-analoge Enzymaktivität abgebaut und damit deaktiviert wird, vergleiche Abb. 1, wodurch die Freisetzung von Glykogen und folglich von Glukose verlangsamt bzw. gestoppt wird. Diese Tatsache war vollkommen überraschend, da man bisher annahm, daß DP IV - wie vorstehend ausgeführt - nur eine Senkung des Blutzuckerspiegels bewirkt.
   Damit eröffnet sich erfindungsgemäß die Möglichkeit, durch Beeinflussung der DP IV-Aktivität bzw. analoger Aktivitäten die Freisetzung endogener Speicherglucose aus Glycogen mittels Glucagon zu befördern; eine gleichzeitige Stimulierung des Glukoseabbaus erfolgt nicht, da im menschlichen Organismus ca. 2 Stunden nach den Mahlzeiten keine Incretine sekretiert werden.

Der Erfindung liegt also der überraschende Befund zugrunde, daß eine Reduktion der im Blutkreislauf agierenden DP IV- oder DP IV-analoger Enzymaktivität kausal zur Beeinflussung des Blutzuckerspiegels führt. Es wurde gefunden, daß
1. die Verminderung von DP IV- bzw. DP IV-analoger Aktivität eine Stabilitätserhöhung von extern zugeführtem oder endogen zirkulierendem Glucagon zur Folge hat, d.h. durch Applikation von Effektoren der DP IV bzw. DP IV-analoger Proteine der Glucagon-Abbau im Blut kontrolliert werden kann;
2. es durch die Stabilitätserhöhung des endogen zirkulierenden oder extern zugeführten Glucagons zu einer kontrollierbaren Modulierung des Blut-Glukosespiegels kommt.

Die Erfindung gemäß Anspruch 1 betrifft somit die Verwendung von Effektoren der Dipeptidyl Peptidase IV (DP IV) - bzw. DP IV-analoger Enzymaktivität zur Herstellung eines Medikaments zur Reduktion von DP IV- bzw. DP IV-analoger Enzymaktivität zur Erhöhung des Blutzuckerspiegels über die für Hypoglycaemie charakteristische Glukosekonzentration im Serum eines Säuger-Organismus hinaus.

Die Erfindung betrifft die Erfindung die Verwendung bzw. Verabreichung von Effektoren der DP IV- bzw. der DP IV-analogen Enzymaktivität an Säuger zur Verhinderung oder Milderung pathologischer Stoffwechsel-Anomalien von Säuger-Organismen, wobei der Blutzuckerspiegel über die für Hypoglycaemie charakteristische Glukosekonzentration im Serum des Säuger-Organismus erhölt wird. Dazu wird dem Säuger-Organismus eine therapeutisch wirksame Menge eines Effektors der DP IV- bzw. der DP IV-analogen Enzymaktivität verabreichen. Eine derartige Anomalie kann z.B. eine akute oder chronische Hypoglycaemie sein, bei der es nötig ist, rasch Kohlehydrat-reserven der Leber zu mobilisieren.

Ein bedeutender Vorteil der vorliegenden Erfindung ist die geringe Belastung des Organismus, da, wenn überhaupt, nur geringe externe Hormongaben verabreicht werden müssen: Erfindungsgemäß wird der Glucagonabbau durch die Verwendung der erfindungsgemäßen DP IV-Inhibitoren gebremst oder vollständig abgestoppt, so daß im Organismus eines erwachsenen Menschen typischerweise eine Menge von verabreichtem oder endogen freigesetztem Glucagon von 2pM - 200 pM erhalten bleibt. Ein zu schneller proteolytischer Abbau wird verhindert.

Die erfindungsgemäß applizierten Effektoren der DP IV- bzw. DP IV-analoger Enzyme können in pharmazeutisch anwendbaren Formulierungskomplexen als Inhibitoren, Substrate, Pseudosubstrate, Inhibitoren der DP IV-Expression, Bindungsproteine oder Antikörper dieser Enzymproteine oder Kombinationen aus diesen verschiedenen Stoffen, die DP IV- bzw. DP IV-analoge Proteinkonzentration im Säugerorganismus reduzieren, zum Einsatz kommen. Erfindungsgemäß eingesetzte Effektoren sind z.B. DP IV-Inhibitoren wie die Dipeptidderivate bzw. Dipeptidmimetika Alanyl-Pyrrolidid, Isoleucyl-Thiazolidid sowie das Pseudosubstrat N-Valyl-Prolyl, O-Benzoyl Hydroxylamin oder deren Salze, insbesondere deren Fumarate. Derartige Verbindungen sind aus der Literatur bekannt oder in Analogie zu den in der Literatur beschriebenen Methoden herstellbar (Demuth, 1990).

Das erfindungsgemäße Verfahren stellt eine neuartige Herangehensweise zur Erhöhung erniedrigter Blutglukosekonzentration im Serum von Säugern dar. Es ist einfach, kommerziell nutzbar und zur Anwendung bei der Therapie, insbesondere von Erkrankungen, die auf unterdurchschnittlichen Blutglukosewerten basieren, in der Humanmedizin geeignet.

Die Effektoren können in Form von pharmazeutischen Präparaten eingesetzt werden, die den Wirkstoff in Kombination mit üblichen aus dem Stand der Technik bekannten Trägermaterialien und/oder üblichen Adjurantien enthalten. Beispielsweise werden sie parenteral (z.B. *i.v.*, in physiologischer Kochsalzlösung) oder enteral (z.B. oral, formuliert mit üblichen Trägermaterialien wie z. B. Glukose) appliziert.

In Abhängigkeit von ihrer endogenen Stabilität und ihrer Bioverfügbarkeit bzw. der Schwere der Erkrankung können einfache oder auch mehrfache Gaben der Effektoren erfolgen, um die erwünschte Normalisierung der Blutglukosewerte zu erreichen. Zum Beispiel kann im Falle von Aminoacyl-Thiazolididen ein solcher Dosisbereich zwischen 0.1 mg und 10 mg Effektorsubstanz pro Kilogramm liegen. Vorzugsweise werden die Effektoren jeweils ca. 120 Min. nach der Nahrungsaufnahme verabreicht. Die Effektoren können auch zusammen bzw. in kurzen zeitlichen Abständen mit Glucagon oder dessen Analogas eingesetzt werden.

### Beispiel 1: Hemmung der Serum-DP IV- katalysierten Glucagonspaltung durch den DP IV-Inhibitor Isoleucyl-Thiazolidid

### Siehe Abbildung 1

### Beispiel 2: Effekt von Glucagon auf die endogene Glukose-freisetzung nach Inkubation in Plasma von DP IVpositiven und DP IV-negativen Ratten

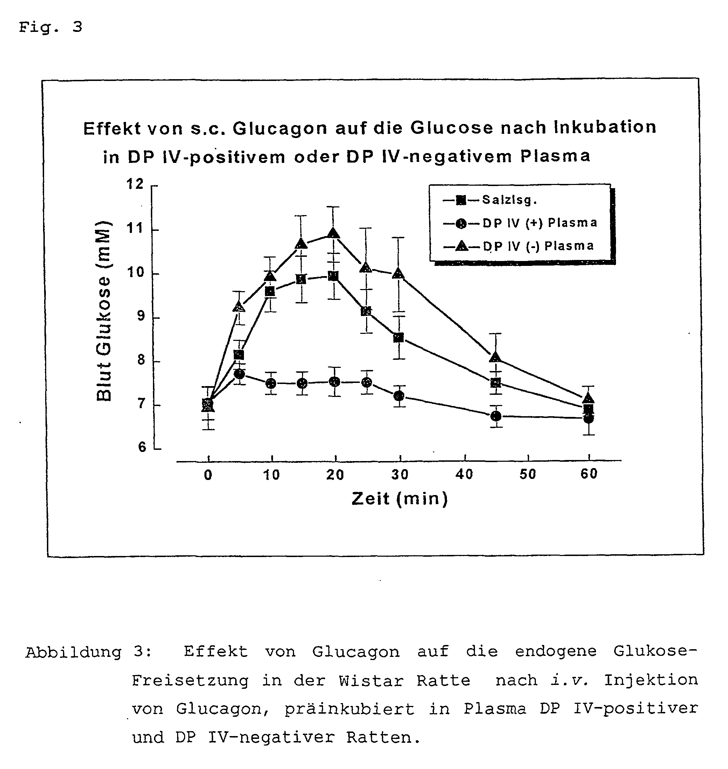

Um zu prüfen, ob die Glucagon-abbauende Aktivität im Plasma von DP IV-negativen Ratten vorhanden ist, wurden 6.8 µg Glucagon für drei Stunden bei 37 °C in 1.0 ml Plasma normaler, DP IV-positiver, Ratten und in 1.0 ml Plasma DP IVnegativer Ratten vorinkubiert. 10 - 50 µl der Inkubationslösung wurden normalen Wistar Ratten *i.v.* injiziert und mit einer Salzkontrolle verglichen. Die biologische Antwort - d.h. die Zunahme der Blutglukose durch die Glucagon-stimulierte hepatische Glukose-Freisetzung wurde 60 min verfolgt (Abbildung 3).

### Beispiel 3: Effekt von Glucagon auf die Glucose Antwort in Wistar Ratten nach i.v. In jek- tion von vorinkubiertem Glucagon im Plasma einer normalen Ratte, in Gegenwart und Abwesenheit von DP IV-Inhibitor

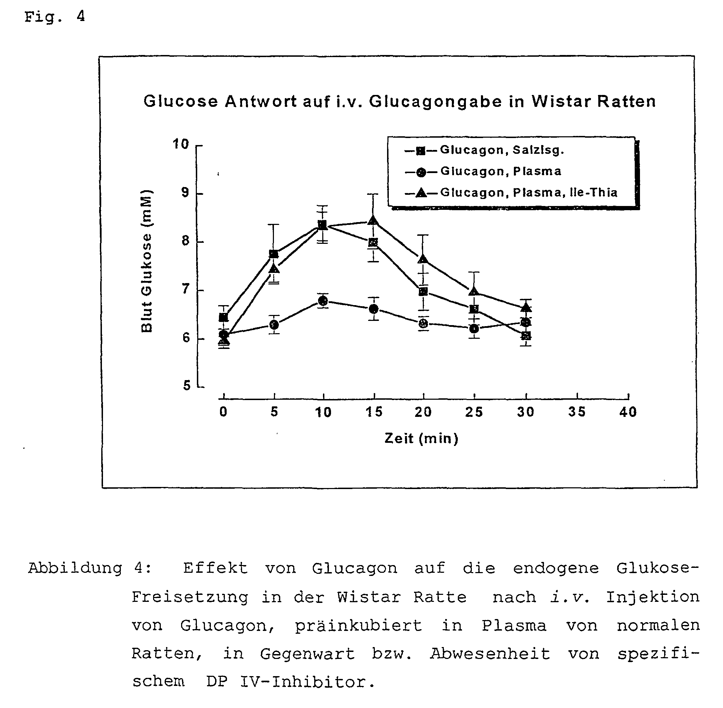

Um zu prüfen, ob der Effekt der Glucagon-abbauenden Aktivität in Plasma durch einen spezifischen DP IV-Inhibitor inhibiert werden kann, wurden 6.8 µg Glucagon für drei Stunden bei 37°C in 1.0 ml normalen Ratten Plasma und in 1.0 ml normalem Ratten-Plasma, das zusätzlich 0,01 mM Isoleucyl-Thiazolidid enthielt, induziert. 10 - 50 µl der Inkubationslösung wurden normalen Wistar Ratten *i.v.* injiziert und mit einer Salzkontrolle verglichen. Die biologische Antwort - d.h. die Zunahme der Blutglukose durch die Glucagon-stimulierte hepatische Glukose-Freisetzung wurde 30 min verfolgt (Abbildung 4).

### Literaturverzeichnis

Ansorge, S., Schön, E., and Kunz, D. (1991). Membrane-bound peptidases of lymphocytes: functional implications. *Biomed. Biochim. Acta* **50**, 799-807.
Demuth, H.-U. (1990) Recent developments in the irreversible inhibition of serine and cysteine proteases. *J*. *Enzyme Inhibition* **3**, 249-280.
Demuth, H.-U., Rosche, F., Schmidt, J., Pauly, R.P., McIntosh, C.H.S. and Pederson, R.A. (1996). Verfahren zur Steigerung des Blutglukosespiegels in Säugern. DE 196 16 486.
Dodge, R.W. & Scheraga, H.A. (1996). Folding and unfolding kinetics of the proline-to-alanine mutants of bovine pancreatic ribonuclease A. *Biochemistry* **35**, 1548-1559.
Gomez, S., Gluschankof, P., Lepage, A., and Cohen, P. (1988).Relationship between endo-and exopeptidases in a processing enzyme system: activation of an endoprotease by the aminopeptidase B-like activity in somatostatin-28 convertase. *Proc Natl Acad Sci U S A* **85,** 5468-5472.
Hegen, M., Niedobitek, G., Klein, C.E., Stein, H., and Fleischer, B. (1990). The T cell triggering molecule Tp 103 is associated with dipeptidyl aminopeptidase IV activity. *J. Immunology* **144,** 2908-2914.
Ishiura, S., Tsukahara, T., Tabira, T., Shimuzu, T., Arahata, K., and Sugita H. (1990). Identification of a putative amyloid A4-generating enzyme as a prolyl endopeptidase *FEBS-Letters* **260**, 131-134.
Kräusslich, H.-G. and Wimmer, E. (1987). Viral Proteinases. *Ann. Rev. Biochem.* **57,** 701
Kessler, H. (1982). Konformation und biologische Wirkung von zyklischen Peptiden. *Angew. Chem.* **94,** 509-520.
Mentlein, R., Gallwitz, B., and Schmidt, W.E. (1993). Dipeptidyl Peptidase IV hydrolyzes gastric inhibitory polypeptide, glucagon-like peptide-1(7-36)amide, peptide histidine methionine and is responsible for their degradation in human serum. *Eur. J. Biochem.* **214**, 829-835.
Pederson, R.A., White, H.A., Schlenzig, D., Pauly, R.P., McIntosh, C.H.S., and Demuth, H.-U. (1998). Improved glucose tolerance in zucker fatty rats treated by oral administration of the dipeptidyl peptidase IV inhibitor isoleucyl thiazolidide. *Diabetes* **47**, 1253-1258.
Vanhoof, G., Goossens, F., De Meester, I., Hendriks, D., and Scharp, S. (1995). Proline motifs and their biological processing. *FASEB Journal* **9**, 736-744.
Walter, R., Simmons, W.H., and Yoshimoto, T. (1980). Proline Specific Endo- and Exopeptidases. *Mol. Cell. Biochem.* **30**, 111-127.
Yaron, A. and Naider, F. (1993). Proline-dependent structural and biological properties of peptides and proteins. *Crit.Rev.Biochem.Mol.Biol.* **28**(1), 31-38.
Kirschke, H., Barrett, A.J., Rawlings, N.D. (1995). Proteinases 1: Lysosomal cysteine proteinases. Protein profile 2/14, S. 1581-1634

## Patentansprüche

1. Verwendung von activitätsmindernden Effektoren der Dipeptidyl Peptidase (DP IV) bzw. DP IV-analoger Enzymaktivität zur Herstellung eines medicaments zur Reduktion von DP IV- bzw. DP IV-analoger Enzymaktivität zur Erhöhung des Blutzuckerspiegels über die für Hypoglycaemie charakteristische Glukosekonzentration im Serum von Säuger Organismen.

2. Verwendung nach Anspruch 1, worin die Reduktion von DP IV- bzw. DP IV-analoger Enzymaktivität in einer erhöhten Stabilität von endogenem oder exogen verabreichtem Glucagon resultiert.

3. Verwendung nach einem der vorstehenden Anspruch, wobei als Effektoren der Dipeptidyl Peptidase (DP IV)- bzw. DP IV-analoger Enzymaktivität Inhibitoren, Substrate, Pseudosubstrate, Inhibitoren der DP IV-Expression, Bindungsproteine oder Antikörper dieser Enzymeproteine oder Kombinationen der genannten Effektoren verwendet werden.

4. Verwendung nach Anspruch 3, worin der effektor der DP IV-und DP IV-anolgen Enzymaktivität ein Inhibitor ist.

5. Verwendung nach einem der vorstehenden Ansprüch, wobei die Effektoren zusammen mit Glucagon oder dessen Analoga eingesetzt werden.

6. Verwendung nach einem der vorstehenden Anspruch, wobei die Effektoren in einer therapeutisch wirksamen Menge eingesetzt werden.

7. Verwendung nach einem der vorstehenden Ansprüch, wobei die Effektoren in Kombination mit an sich üblichen Adjuvantien und/oder Trägerstoffen eingesetzt werden.

## Claims

1. Use of activity-reducing effectors of dipeptidyl peptidase (DP IV) or DP IV-analogous enzymic activity for producing a medicament for reducing DP IV or DP IV-analogous enzymic activity for elevating the blood glucose level above the glucose concentration characteristic of hypoglycaemia in the serum of a mammalian organism.

2. Use according to Claim 1, in which the reduction of DP IV or DP IV-analogous enzymic activity results in an increased stability of endogenous or exogenously administered glucagon.

3. Use according to either of the preceding claims, where the effectors of dipeptidyl peptidase (DP IV) or DP IV-analogous enzymic activity used are inhibitors, substrates, pseudosubstrates, inhibitors of DP IV expression, binding proteins or antibodies of these enzymic proteins or combinations of the said effectors.

4. Use according to Claim 3, in which the effector of DP IV and DP IV-analogous enzymic activity is an inhibitor.

5. Use according to any of the preceding claim, where the effectors are employed together with glucagon or its analogues.

6. Use according to any of the preceding claim, where the effectors are employed in a therapeutically effective amount.

7. Use according to any of the preceding claim, where the effectors are employed in combination with adjuvants and/or carriers which are conventional per se.

## Revendications

1. Utilisation d'effecteurs diminuant l'activité de la dipeptidyl-peptidase (DP IV) ou l'activité enzymatique analogue à la DP IV pour la préparation d'un médicament destiné à réduction de la DP IV ou de l'activité enzymatique analogue à la DP IV en vue d'augmenter le niveau de sucre dans le sang au-dessus de la concentration en glucose caractéristique de l'hypoglycémie dans le sérum d'organismes de mammifères.

2. Utilisation selon la revendication 1, dans laquelle la réduction de la DP IV ou de l'activité enzymatique analogue à la DP IV résulte en une stabilité plus élevée du glucagon endogène ou administré par voie exogène.

3. Utilisation selon l'une quelconque des revendications précédentes, dans laquelle on utilise comme effecteurs de la dipeptidyl-peptidase (DP IV) ou de l'activité enzymatique analogue à la DP IV des inhibiteurs, des substrats, des pseudo-substrats, des inhibiteurs de l'expression de la DP IV, des protéines de liaison ou des anticorps de ces protéines enzymatiques ou des combinaisons des effecteurs mentionnés.

4. Utilisation selon la revendication 3, dans laquelle l'effecteur de la DP IV et de l'activité enzymatique analogue à la DP IV est un inhibiteur.

5. Utilisation selon l'une quelconque des revendications précédentes, les effecteurs étant utilisés avec le glucagon ou ses analogues.

6. Utilisation selon l'une quelconque des revendications précédentes, les effecteurs étant utilisés en une quantité thérapeutiquement active.

7. Utilisation selon l'une quelconque des revendications précédentes, les effecteurs étant utilisés en combinaison avec des adjuvants et/ou des substances support usuels en soi.
